# FASCICULE DE BREVET EUROPEEN

(11) **EP 4 341 850 B1**
(45) Date de publication et mention de la délivrance du brevet: **21.01.2026**
(21) Numéro de dépôt: 22730365.8
(22) Date de dépôt: 16.05.2022
(51) Int. Cl.: G06V 20/20, A61B 90/90

(54) **PROCÉDÉ D'IDENTIFICATION D'INSTRUMENTS MÉDICAUX**
VERFAHREN ZUR IDENTIFIZIERUNG VON MEDIZINISCHEN VORRICHTUNGEN
METHOD FOR IDENTIFYING MEDICAL DEVICES

(30) Priorité: 17.05.2021 FR 2105108
(43) Date de publication de la demande: 27.03.2024
(73) Titulaire: Vygon, 95440 Ecouen (FR)
(72) Inventeur: WALTER, Thomas, 92500 RUEIL MALMAISON (FR); TENAILLEAU, Tiphaine, 95110 SANNOIS (FR); LAFOESTE, Marie, 93200 SAINT DENIS (FR); DAILLY, Emi, 95340 PERSAN (FR)
(74) Mandataire: Lavoix
(86) Numéro de dépôt international: PCT/EP2022/063203
(87) Numéro de publication internationale: WO 2022/243251

(56) Documents cités:
- EP-A1- 3 307 136
- US-A1- 2015 209 114
- LELACHAICHAROEANPAN JAROONWIT ET AL: "Classification of Surgical Devices with Artificial Neural Network Approach", 2021 7TH INTERNATIONAL CONFERENCE ON ENGINEERING, APPLIED SCIENCES AND TECHNOLOGY (ICEAST), IEEE, 1 April 2021 (2021-04-01), pages 154 - 159, XP033914190, DOI: 10.1109/ICEAST52143.2021.9426258

## Description

La présente invention concerne un procédé d'identification d'instruments médicaux. La présente invention concerne aussi un produit programme d'ordinateur associé.

Différents instruments médicaux sont utilisables pour la mise d'un patient sous perfusion. De tels dispositifs comprennent généralement une partie interne destinée à être insérée dans le patient, par exemple dans une veine, une artère, l'œsophage (ou autre partie du système digestif), le liquide cephalorachidien (cathéters périduraux) ou dans un dispositif sous-cutané (ex : chambre implantable), et une partie externe visible de l'extérieur et destinée à être manipulée par le personnel de santé, par exemple, pour l'injection de solutions médicamenteuses ou de nutrition.

Toutefois, il n'est pas toujours aisé pour le personnel de santé d'identifier la nature d'un instrument médical lorsque celui-ci est déjà implanté dans le patient. Or, une mauvaise identification est susceptible d'engendrer des effets secondaires graves pour le patient, de par le non-respect des conditions d'utilisation de l'instrument médical ou l'injection d'un produit inadapté.

En particulier, un tel risque de confusion est accru dans le cas des cathéters PICC (cathéter veineux central d'insertion périphérique) et cathéters Midlines (cathéter veineux périphérique) puisque de tels cathéters émergent tous deux au niveau du bras des patients. Les risques de confusion sont notamment décrits dans l'article Letournel C. Gestion du risque de confusion entre PICC Lines et Midlines. J Pharm Clin 2018 ; 37(1) : 19-26 doi: 10.1684/jpc.2017.0374. La confusion entre PICC et Midlines est notamment susceptible d'engendrer un risque d'injection de médicaments veinotoxiques par un cathéter Midline ou un risque d'infection si la durée maximale d'utilisation n'est pas respectée.

En outre, il apparaît que les spécificités d'utilisation, ainsi que les bonnes pratiques d'utilisation (Recommandations SF2H : Prévention des infections liées aux cathéters périphériques vasculaires et sous cutanés. Mai 2019, Hygiènes Vol XXVII - n°2 ; Infusion Therapy Standards of Practice 8th Edition, Jan/Feb 2021, Journal of Infusion Nursing, vol 44, n°51) des différents instruments médicaux, qu'ils soient introduits dans le corps, en contact avec la peau, ou connectés à des instruments introduits dans le corps ou en contact avec la peau, ne sont pas toujours bien connues du personnel de santé. Cela est également susceptible d'engendrer des complications pour le patient dues à une mauvaise utilisation de ces instruments.

US 2015/209114 A divulgue un système médical automatisé capable de détecter des événements ou conditions cliniques à l'aide de capteurs intégrés à des instruments médicaux, et d'afficher en réponse des instructions contextuelles sur un dispositif de visualisation.

Il existe donc un besoin pour la mise en place de moyens permettant de diminuer les risques de mauvaise identification, par le personnel de santé, d'instruments médicaux portés par un patient, et par là-même de diminuer les risques de mésusage ou de non-respect des bonnes pratiques d'utilisation de tels instruments.

A cet effet, la présente description a pour objet un procédé d'identification d'instruments médicaux, dit instruments médicaux d'intérêt, les instruments médicaux d'intérêt étant des instruments destinés à être introduits dans le corps d'un patient ou à être mis en contact avec le corps d'un patient, ou étant des instruments destinés à être connectés à des instruments introduits dans le corps d'un patient ou en contact avec le corps d'un patient, chaque instrument médical d'intérêt ayant une partie externe à l'extérieur du corps du patient, le procédé comprenant les étapes suivantes :
- la collection d'images de parties externes d'instruments médicaux d'intérêt, chaque image étant labellisée avec un identifiant de l'instrument médical d'intérêt parmi un ensemble d'identifiants prédéfinis, les images collectées formant une base d'images d'entraînement, l'étape de collection étant mise en œuvre par ordinateur,
- l'entraînement d'un modèle d'identification à partir de la base d'images d'entraînement pour obtenir un modèle d'identification entraîné pour attribuer un identifiant, parmi l'ensemble d'identifiants prédéfinis, à un instrument médical d'intérêt à identifier en fonction d'une image de la partie externe de l'instrument médical, l'étape d'entraînement étant mise en œuvre par ordinateur,
- la réception d'une image de la partie externe d'un instrument médical d'intérêt à identifier, l'étape de réception étant mise en œuvre par ordinateur,
- l'attribution, par le modèle de classification entraîné, d'un identifiant à l'instrument médical d'intérêt imagé sur l'image reçu, l'étape de classification étant mise en œuvre par ordinateur, et
   la caractérisation de l'instrument médical d'intérêt en fonction de l'identifiant attribué, dans lequel chaque identifiant est associé à un lien internet et/ou à une base de données à distance par lequel/laquelle il est obtenu un support visuel relatif à l'utilisation de l'instrument médical d'intérêt, l'étape de caractérisation comprenant l'affichage du support visuel correspondant au lien internet ou à la base de données associé(e) à l'identifiant attribué, le support visuel étant par exemple une vidéo, une image ou un article.

Suivant des modes de réalisation particuliers, le procédé comprend une ou plusieurs des caractéristiques suivantes, prise(s) isolément ou suivant toutes les combinaisons techniquement possibles :
- le procédé comprend une étape de manipulation de l'instrument médical d'intérêt en fonction de la caractérisation effectuée.
- la base d'images d'entraînement est formée d'images d'instruments médicaux d'intérêt d'au moins deux natures différentes, les au moins deux natures d'instruments médicaux sur les images de la base d'images d'entraînement, étant choisies parmi les natures suivantes : tube médical, cathéter veineux, cathéter artériel, cathéter de nutrition entéral, cathéter de nutrition parentéral, cathéter de drainage, cathéter péridural, cathéter systémique, cathéter veineux central à insertion périphérique, dit PICC, cathéter veineux périphérique, dit Midline, cathéter veineux central, dit CVC, cathéter ombilical, aiguille de Huber, système de fixation cutanée de tubes médicaux, pansement de recouvrement, valve, système clos et bouchon connecté à une embase d'un tube médical.
- chaque identifiant est associé à une donnée relative à une nature d'instrument médical, l'étape de caractérisation comprenant la détermination de la nature de l'instrument médical d'intérêt imagé sur l'image reçue en fonction de l'identifiant attribué.
- chaque identifiant est associé à des recommandations d'utilisation de l'instrument médical d'intérêt, l'étape de caractérisation comprenant la détermination des recommandations d'utilisation de l'instrument médical d'intérêt imagé sur l'image reçue en fonction de l'identifiant attribué.
- les recommandations d'utilisation comprennent une ou plusieurs des recommandations suivantes :
   o la voie d'abord à utiliser pour l'introduction de l'instrument médical d'intérêt,
   o le débit maximal d'injection de liquide dans l'instrument médical d'intérêt,
   o la pression maximale de mise en défaut de l'instrument médical d'intérêt,
   o la fréquence de changement d'un éventuel pansement appliqué sur l'instrument médical d'intérêt,
   o la compatibilité de l'instrument médical d'intérêt avec d'autres types d'instruments médicaux,
   o la compatibilité de l'instrument médical d'intérêt avec des solutions médicamenteuses, et
   o la compatibilité de l'instrument médical d'intérêt avec des examens médicaux prédéfinis, et le cas échéant, les conditions de réalisation des examens.
- chaque identifiant est associé à au moins une caractéristique structurelle de l'instrument médical d'intérêt, la ou au moins une caractéristique structurelle étant choisie parmi : la marque de l'instrument médical d'intérêt, le contact du fabricant légal de l'instrument médical d'intérêt, le ou les matériaux dans lesquels est réalisé l'instrument médical d'intérêt, le ou les éventuels matériaux potentiellement allergènes dans lesquels est réalisé l'instrument médical d'intérêt, la taille de l'instrument médical d'intérêt et, lorsque l'instrument médical d'intérêt est un cathéter ou un tube médical, le diamètre et la longueur initiale du cathéter ou du tube médical, le nombre de lumières du cathéter ou du tube médical, le volume mort de chaque lumière du cathéter ou du tube médical, les débits, par gravité ou par injection via une pompe ou un injecteur de produits de contraste, du cathéter ou du tube médical, l'étape de caractérisation comprenant la détermination de la ou des caractéristiques structurelles de l'instrument médical d'intérêt imagé sur l'image reçue en fonction de l'identifiant attribué.
- chaque instrument médical d'intérêt présente une partie interne connectée à la partie externe, la partie interne étant un tube ou une aiguille introduite dans le corps du patient, la partie externe comprenant au moins un tube et/ou à un connecteur à un tube.
- l'identifiant attribué à l'instrument médical d'intérêt est associé à une base de données à distance par laquelle il est obtenu des informations d'utilisation de l'instrument médical.
- les informations d'utilisation comprennent des données définies par le fabricant et/ou des données définies par un professionnel de santé lors de l'utilisation de l'instrument médical sur un patient.
- le procédé comprend une étape de mise à jour des informations d'utilisation de l'instrument médical pour un patient donné et/ou une utilisation donnée, les informations d'utilisation étant archivées de sorte à assurer un suivi du patient et/ou une traçabilité de l'instrument médical.
- l'identifiant est associé à une information relative à l'impact environnemental et/ou à l'écoconception de l'instrument médical d'intérêt.

La présente description se rapporte également à un produit programme d'ordinateur comprenant des instructions de programme enregistrées sur un support lisible par ordinateur, pour l'exécution d'un procédé tel que décrit précédemment lorsque le programme d'ordinateur est exécuté sur un ordinateur.

La présente description concerne aussi un support lisible d'informations sur lequel est mémorisé un produit programme d'ordinateur tel que précédemment décrit.

D'autres caractéristiques et avantages de l'invention apparaîtront à la lecture de la description qui suit de modes de réalisation de l'invention, donnés à titre d'exemple uniquement et en référence aux dessins qui sont :
[Fig 1], Figure 1, une vue schématique d'un exemple d'ordinateur permettant la mise en œuvre d'un procédé d'identification d'instruments médicaux,
[Fig 2], Figure 2, un organigramme d'un exemple de mise en œuvre d'un procédé d'identification d'instruments médicaux,
[Fig 3], Figure 3, une représentation schématique d'un exemple d'un cathéter,
[fig 4], Figure 4, une représentation schématique d'un exemple de tube médical,
[Fig 5], Figure 5, une représentation schématique d'un exemple d'une aiguille de Huber sécurisée,
[Fig 6], Figure 6, représentation schématique d'un exemple d'un instrument médical en contact avec la peau d'un patient (pansement transparent recouvrant le site d'insertion cutanée d'un cathéter),
[Fig 7], Figure 7, représentation schématique d'un autre exemple d'un instrument médical en contact avec la peau d'un patient (pansement de fixation d'un cathéter), et
[Fig 8], Figure 8, représentation schématique d'un exemple de système clos connecté à l'embase d'un cathéter.

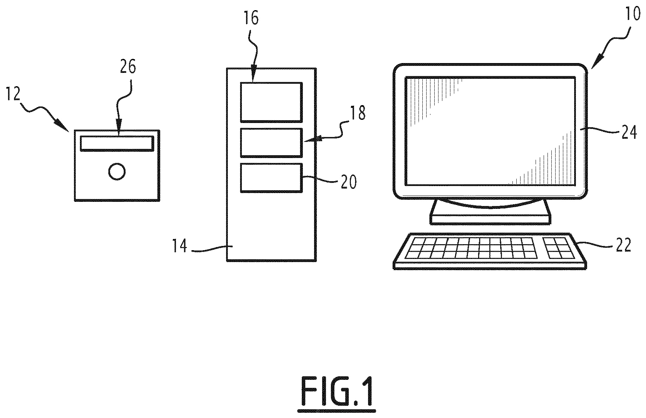

Un calculateur 10 et un produit programme d'ordinateur 12 sont illustrés par la figure 1.

Le calculateur 10, est de préférence, un ordinateur.

Plus généralement, le calculateur 10 est un calculateur électronique propre à manipuler et/ou transformer des données représentées comme des quantités électroniques ou physiques dans des registres de calculateur 10 et/ou des mémoires en d'autres données similaires correspondant à des données physiques dans des mémoires, des registres ou d'autres types de dispositifs d'affichage, de transmission ou de mémorisation.

Le calculateur 10 est en interaction avec le produit programme d'ordinateur 12.

Comme illustré par la figure 1, le calculateur 10 comporte un processeur 14 comprenant une unité de traitement de données 16, des mémoires 18 et un lecteur 20 de support d'informations. Dans l'exemple illustré par la figure 1, le calculateur 10 comprend un clavier 22 et une unité d'affichage 24.

Le produit programme d'ordinateur 12 comporte un support d'informations 26.

Le support d'information 26 est un support lisible par le calculateur 10, usuellement par l'unité de traitement de données 16. Le support lisible d'informations 26 est un médium adapté à mémoriser des instructions électroniques et capable d'être couplé à un bus d'un système informatique.

A titre d'exemple, le support d'informations 26 est une disquette ou disque souple (de la dénomination anglaise « *Floppy disc* »), un disque optique, un CD-ROM, un disque magnéto-optique, une mémoire ROM, une mémoire RAM, une mémoire EPROM, une mémoire EEPROM, une carte magnétique ou une carte optique.

Sur le support d'informations 26 est mémorisé le programme d'ordinateur 12 comprenant des instructions de programme.

Le programme d'ordinateur 12 est chargeable sur l'unité de traitement de données 16 et est adapté pour entraîner la mise en œuvre d'un procédé d'identification d'instruments médicaux, lorsque le programme d'ordinateur 12 est mis en œuvre sur l'unité de traitement 16 du calculateur 10.

Bien que la figure 1, illustre un calculateur sous la forme d'un ordinateur physique, la présente description ne se limite pas à un tel type ordinateur. Elle s'étend à tout dispositif intégrant un calculateur en lien avec un programme d'ordinateur (application informatique).

Ainsi, dans un exemple de réalisation, le calculateur et le produit programme d'ordinateur 12 sont intégrés dans un smartphone (ordiphone), une tablette tactile ou tout autre objet connecté.

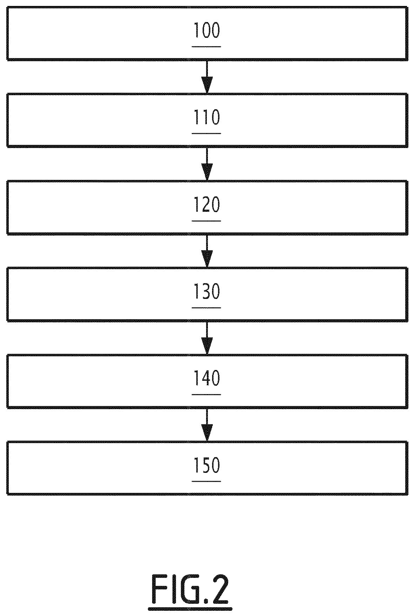

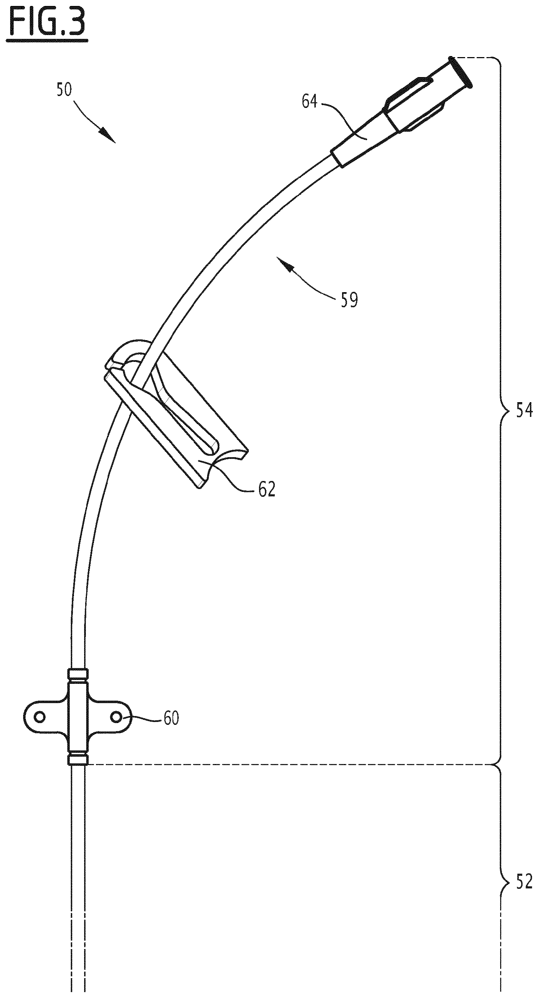

Le fonctionnement du calculateur 10 va maintenant être décrit en référence à l'exemple de la figure 2, qui illustre un organigramme d'un procédé d'identification d'instruments médicaux, et aux figures 3 à 8 qui illustrent des exemples d'instruments médicaux considérés dans le cadre du présent procédé.

Les instruments médicaux considérés dans le cadre du présent procédé sont dits instruments médicaux d'intérêt 50.

Les instruments médicaux d'intérêt 50 comprennent, par exemple, une partie interne 52 et une partie externe 54 connectée à la partie interne 52.

La partie interne 52 est destinée à être introduite dans le corps du patient. Lorsque l'instrument médical 50 est déjà introduit dans le corps du patient, la partie interne 52 est, ainsi, une partie non visible du personnel de santé. La partie interne 52 est typiquement un tube ou une aiguille.

La partie externe 54 est destinée à rester à l'extérieur du corps du patient de sorte à être manipulée par le personnel de santé, par exemple, pour l'injection de médicaments. La partie externe 54 comprend au moins un tube et/ou un connecteur à un tube.

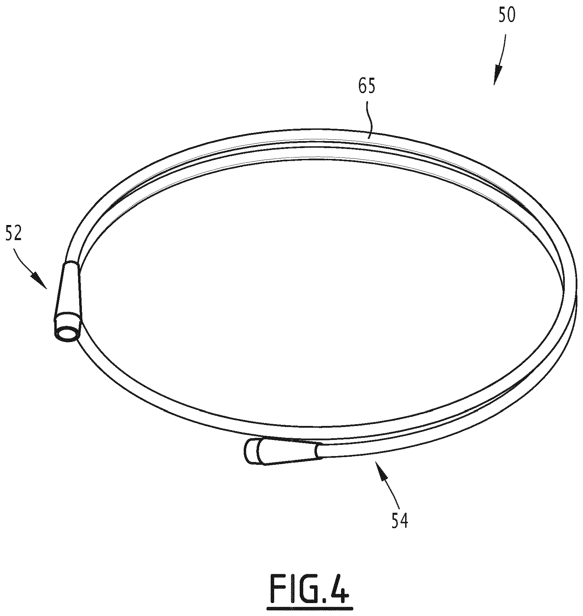

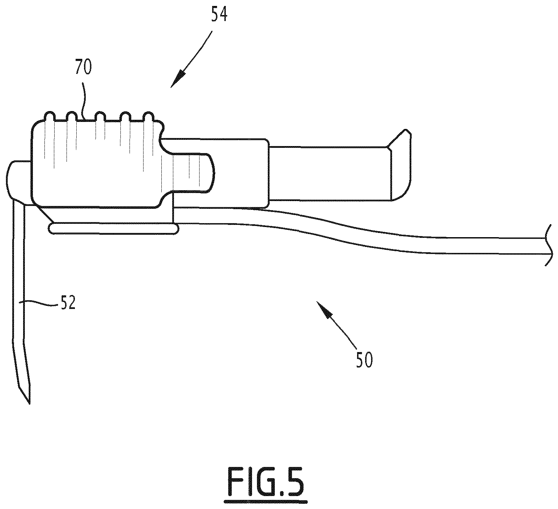

Les instruments médicaux d'intérêt 50 considérés ayant une partie interne 52 connectée à une partie externe 54 sont, par exemple, des cathéters, tunnelisés ou non (illustrés en figure 3), plus largement des tubes médicaux (illustrés en figure 4), et/ou des aiguilles de Huber (illustrées en figure 5).

Comme illustré par la figure 3, la partie externe 54 d'un cathéter comprend un tube 59 avec généralement une embase 60, une pince 62 (appelée clamp) et un connecteur 64 (luer lock). Une valve (non représentée) est généralement insérée au bout du connecteur 64. L'embase 60 est généralement recouverte d'un système de fixation adhésif ou par ancrage sous-cutané, et d'un pansement transparent.

Les cathéters sont, par exemple, des cathéters veineux, des cathéters artériels, des cathéters de nutrition entérale/parentéral, des cathéters de drainage, des cathéters périduraux, cathéters systémiques ou encore des cathéters intraveineux. Il est clairement établi (cf. Outils de Sécurisation et d'auto-évaluation de l'administration des médicaments, HAS, mai 2013 ; Norme ISO 80369-1 :2018) que pour tous ces cathéters et tubes médicaux, il existe une problématique de confusion des différentes voies d'abord : notamment central vs périphérique, entéral vs parentéral, pulmonaire vs autres voies et intrathécal vs intravéneux. Les voies d'abord multiples, de même que la non reconnaissance du cathéter ou tube médical, peuvent être à l'origine d'erreurs parfois graves. Par exemple, l'injection de vinca-alcaloïdes, fortement neurotoxiques par voie intra-thécale, est potentiellement mortelle pour le patient. De plus, l'injection de molécules classifiés comme vesicantes (S. Manrique-Rodriguez, Standardization and Chemical Characterization of Intravenous Therapy in Adult Patients : A Step Further in Medication Safety, Drugs in R&D, December 2020) dans un cathéter périphérique non approprié peut entraîner des risques de veinite, nécrose voire extravasation.

Plus spécifiquement, les cathéters sont, par exemple, des cathéters veineux centraux à insertion périphérique, dits PICC, des cathéters veineux périphériques, dits Midlines et des cathéters veineux centraux, dits CVC, des cathéters ombilicaux centraux, ou des cathéters de nutrition entérale.

Un cathéter PICC est un cathéter veineux destiné à être inséré au-dessus du pli du coude dans une veine profonde du bras jusqu'à la jonction cavo-atriale. L'extrémité distale est donc située à l'entrée du cœur. Un PICC est notamment utilisable pour les injections suivantes : chimiothérapie, antibiothérapie, alimentation parentérale et transfusion. Un PICC peut rester implanté jusqu'à 6 mois dans un patient.

Un cathéter Midline est un cathéter destiné à être inséré au-dessus du pli du coude dans une veine du bras jusqu'à la ligne axillaire. L'extrémité distale est donc située en-dessous de la clavicule. Un Midline est notamment utilisable pour les injections de produits non irritants (< 900 mOs/L, 5<pH<9) en traitement prolongé (supérieur à 8 jours), tels que des antibiotiques, analgésiques ou produits d'hydratation. Un Midline peut rester implanté jusqu'à 30 jours dans un patient.

Un cathéter CVC est un cathéter destiné à être inséré au niveau du cou, du thorax ou au niveau fémoral. L'extrémité distale d'un tel cathéter est située à l'entrée du cœur.

Un autre type de tube est illustré par la figure 4. Dans l'exemple illustré par la figure 4, l'instrument médical 50 est un tube 65 dont une extrémité est destinée à être insérée dans le patient et forme ainsi la partie interne 52, et dont l'autre extrémité forme la partie externe 54. Dans l'exemple particulier de la figure 4, le tube est de type tubulure à oxygène. Toutefois, l'instrument médical 50 est potentiellement tout autre type de tube.

Une aiguille de Huber est un instrument destiné à être utilisé sur une chambre à cathéter implantable, dite CCI. La chambre CCI est un dispositif implantable sous-cutané composé d'une chambre d'injection et d'un cathéter central. L'aiguille de Huber permet les injections et prélèvements en ponctionnant le boîtier de la chambre sous la peau. L'aiguille de Huber comprend notamment une aiguille (à biseau tangentielle) destinée à être insérée dans la peau du patient au niveau du boîtier et un connecteur destiné à rester à l'extérieur de sorte à être connecté à un tube par exemple.

Comme illustré par la figure 5, la partie interne 52 d'une aiguille de Huber est une aiguille (à biseau tangentielle) destinée à être insérée dans la peau du patient au niveau du boîtier. La partie externe 54 comprend un connecteur 70 destiné à rester à l'extérieur de sorte à être connecté à un tube par exemple.

En complément ou en variante, les ou des instruments médicaux d'intérêt 50 sont constitués seulement d'une partie externe 54 visible depuis l'extérieur du patient (pas de partie interne introduite dans le corps).

Dans ce cas, la partie externe 54 est, par exemple, destinée à être en contact avec la peau du patient ou à être connectée à un instrument médical destiné à être introduit dans le corps d'un patient ou en contact avec la peau du patient.

Les instruments médicaux d'intérêt 50 considérés ayant seulement une partie externe 54 sont, par exemple, des systèmes cutanés du type : système de fixation cutanée de tubes médicaux (cathéters, drains, etc.), pansement de recouvrement, valves, système clos et bouchons connectés à l'embase d'un tube médical (embase 60 d'un cathéter par exemple).

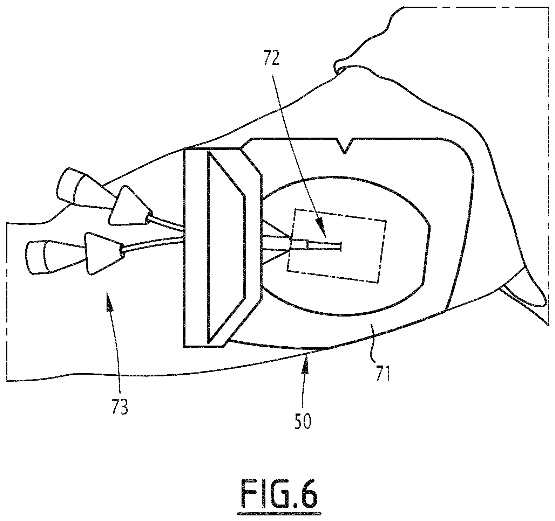

La figure 6 illustre à titre d'exemple un instrument médical d'intérêt 50 en contact avec la peau d'un patient. Dans cet exemple, l'instrument médical 50 est un pansement transparent 71 recouvrant le site 72 d'insertion cutanée d'un cathéter 73.

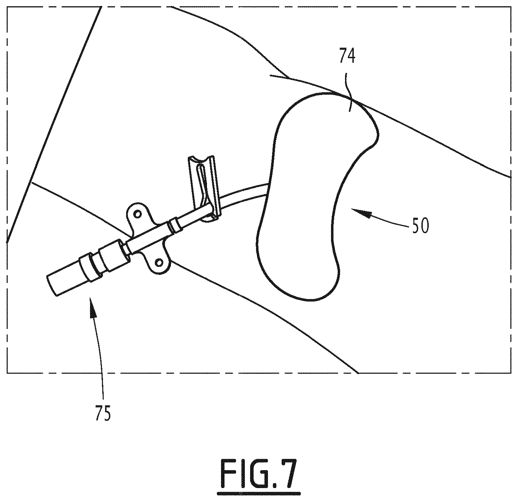

La figure 7 est un autre exemple d'un instrument médical d'intérêt 50 en contact avec la peau d'un patient. Dans cet exemple, l'instrument médical 50 est un pansement 74 de fixation d'un cathéter 75.

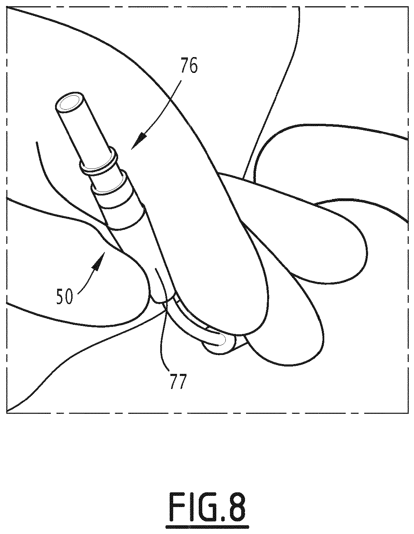

La figure 8 est encore un autre exemple d'un instrument médical d'intérêt 50 connecté à un instrument médical destiné à être introduit dans le corps d'un patient ou en contact avec la peau du patient. Dans cet exemple, l'instrument médical 50 est un système clos 76 connecté à l'embase 77 d'un cathéter.

Le procédé d'identification comprend une étape 100 de collection d'images de parties externes d'instruments médicaux d'intérêt 50. Les images collectées forment, ainsi, une base d'images d'entraînement. L'étape 100 est mise en œuvre par le calculateur 10 en interaction avec le produit programme d'ordinateur 12, c'est-à-dire est mise en œuvre par ordinateur.

Les images de la base de données ont, par exemple, été acquises par une caméra, par exemple, appartenant à un smartphone.

De préférence, les images collectées sont des images sur lesquelles les instruments médicaux d'intérêt 50 sont en configuration d'utilisation, c'est-à-dire que lorsque les instruments 50 comprennent une partie interne 52, la partie interne est introduite dans le corps du patient, et lorsque les instruments 50 comprennent seulement une partie externe 54, la partie externe 54 est en contact avec la peau du patient ou connectée à un autre instrument médical introduit dans le corps ou en contact avec la peau du patient. Seules les parties externes des instruments médicaux sont, ainsi, visibles sur les images.

En variante, les images collectées comprennent aussi des images d'instruments médicaux d'intérêt 50 qui ne sont pas portés par le patient.

La base d'images d'entraînement est typiquement formée d'images d'instruments médicaux d'intérêt 50 d'au moins deux natures différentes.

Dans un exemple, les au moins deux natures d'instruments médicaux 50 imagés sur les images de la base d'images d'entraînement, sont choisies parmi les natures suivantes : tube médical, cathéter veineux, cathéter artériel, cathéter de nutrition entéral, cathéter de nutrition parentéral, cathéter de drainage, cathéter péridural, cathéter systémique, cathéter veineux central à insertion périphérique, dit PICC, cathéter veineux périphérique, dit Midline, cathéter veineux central, dit CVC, cathéter ombilical, aiguille de Huber, système de fixation cutanée de tubes médicaux (cathéters, drains, etc.), pansement de recouvrement, valve, système clos et bouchon connecté à une embase d'un tube médical.

Dans un exemple spécifique, au moins une nature d'instrument imagé est un cathéter veineux central à insertion périphérique, dit PICC, et au moins une nature d'instrument imagé est un cathéter veineux périphérique, dit Midline, de sorte à différencier ces deux types de cathéters.

Chaque image est labellisée avec un identifiant de l'instrument médical d'intérêt 50 parmi un ensemble d'identifiants prédéfinis. L'identifiant a, par exemple, été déterminé par un opérateur.

Chaque identifiant prédéfini correspond à une même catégorie d'instruments médicaux, voire à un modèle spécifique d'instrument médical (même référence). Les instruments médicaux labellisés avec le même identifiant ont donc des caractéristiques communes ou correspondent aux mêmes instruments.

Avantageusement, chaque identifiant est associé à une donnée relative à une nature d'instrument médical. Les natures d'instruments médicaux sont, par exemple, une ou plusieurs des natures suivantes : tube médical, cathéter veineux, cathéter artériel, cathéter de nutrition entéral, cathéter de nutrition parentéral, cathéter de drainage, cathéter péridural, cathéter systémique, cathéter veineux central à insertion périphérique, dit PICC, cathéter veineux périphérique, dit Midline, cathéter veineux central, dit CVC, cathéter ombilical, aiguille de Huber, système de fixation cutanée de tubes médicaux (cathéters, drains, etc.), pansement de recouvrement, valve, système clos et bouchon connecté à une embase d'un tube médical.

Dans un exemple spécifique, au moins une nature d'instrument est un cathéter veineux central à insertion périphérique, dit PICC, et au moins une nature d'instrument est un cathéter veineux périphérique, dit Midline, de sorte à différencier ces deux types de cathéters.

Avantageusement, chaque identifiant est associé à des recommandations d'utilisation de l'instrument médical d'intérêt 50. Les recommandations visent à guider le personnel de santé dans la bonne utilisation des instruments.

Dans un exemple, les recommandations d'utilisation comprennent une ou plusieurs des recommandations suivantes :
a. la voie d'abord à utiliser (périphérique, centrale, entérale etc.),
b. le débit maximal d'injection de liquide (par gravité et via une pompe ou un injecteur de produit de contraste) dans l'instrument médical d'intérêt 50,
c. la pression maximale de mise en défaut de l'instrument médical d'intérêt 50,
d. la fréquence de changement d'un éventuel pansement appliqué sur l'instrument médical d'intérêt 50,
e. la compatibilité de l'instrument médical d'intérêt 50 avec d'autres types d'instruments médicaux,
f. la compatibilité de l'instrument médical d'intérêt 50 avec des solutions médicamenteuses, et
g. la compatibilité de l'instrument médical d'intérêt 50 avec des examens médicaux prédéfinis, et le cas échéant, les conditions de réalisation des examens.

Avantageusement, chaque identifiant est associé à un lien internet et/ou à une base de données à distance par lequel/laquelle il est obtenu un support visuel relatif à l'utilisation de l'instrument médical d'intérêt 50. Le support visuel est destiné à être visualisé ou lu par le personnel de santé pour aider à la bonne manipulation de l'instrument médical d'intérêt 50. Le support visuel est par exemple une vidéo, une image ou un article.

Avantageusement, chaque identifiant est associé à des caractéristiques structurelles de l'instrument médical d'intérêt 50. Les caractéristiques structurelles comprennent, par exemple, une ou plusieurs des caractéristiques suivantes : la marque de l'instrument médical d'intérêt 50, le contact du fabricant légal de l'instrument médical d'intérêt 50, le ou les matériaux dans lesquels est réalisé l'instrument médical d'intérêt 50, le ou les éventuels matériaux potentiellement allergènes dans lesquels est réalisé l'instrument médical d'intérêt 50, la taille de l'instrument médical d'intérêt 50 et, lorsque l'instrument médical d'intérêt 50 est un cathéter ou un tube médical, le diamètre et la longueur initiale du cathéter ou du tube médical, le nombre de lumières du cathéter ou du tube médical, le volume mort de chaque lumière (voie) du cathéter ou du tube médical, les débits, par gravité ou par injection via une pompe ou un injecteur de produits de contraste, du cathéter ou du tube médical.

Le procédé d'identification comprend une étape 110 d'entraînement d'un modèle d'identification à partir de la base d'images d'entraînement. L'étape 110 permet d'obtenir un modèle d'identification entraîné pour attribuer un identifiant, parmi l'ensemble d'identifiants prédéfinis, à un instrument médical d'intérêt 50 à identifier en fonction d'une image de la partie externe 54 de l'instrument médical d'intérêt 50. L'étape 100 est mise en œuvre par le calculateur 10 en interaction avec le produit programme d'ordinateur 12, c'est-à-dire est mise en œuvre par ordinateur.

Dans un exemple de mise en œuvre, l'entraînement du modèle d'identification est réalisé selon une technique d'apprentissage appliquée à la base de données.

Le modèle d'identification est, par exemple, un réseau de neurones. La technique d'apprentissage permet de configurer le réseau de neurones au fur et à mesure de son apprentissage réalisé sur la base de données. Dans un exemple, une partie de la base de données est utilisée pour configurer le réseau de neurones et l'autre partie pour valider la configuration.

Typiquement, le modèle entraîné est propre à d'abord reconnaître le modèle spécifique (référence) de l'instrument médical, correspondant à un identifiant, via des caractéristiques physiques propres à ce modèle d'instrument. Les caractéristiques physiques sont, par exemple, la couleur, la forme ou des accessoires de l'instrument médical. Les autres caractéristiques de l'instrument sont ensuite retrouvées en fonction de l'identifiant (ex : la nature de l'instrument, les recommandations d'utilisation...).

Le procédé d'identification comprend une étape 120 de réception d'une image de la partie externe 54 d'un instrument médical d'intérêt 50 à identifier. L'étape de réception 120 est mise en œuvre par le calculateur 10 en interaction avec le produit programme d'ordinateur 12, c'est-à-dire est mise en œuvre par ordinateur.

L'instrument médical d'intérêt 50 imagé est, par exemple, en condition d'utilisation sur le patient. En variante, l'instrument médical d'intérêt 50 imagé n'est pas porté par le patient.

L'image reçue a, par exemple, été acquise par la caméra d'un smartphone. L'acquisition a, par exemple, été déclenchée par du personnel de santé en vue d'identifier un instrument médical d'intérêt 50, par exemple un ou plusieurs instruments médicaux d'intérêt 50 introduit dans le corps d'un patient ou en contact avec sa peau.

Le procédé d'identification comprend une étape 130 d'attribution, par le modèle de classification entraîné, d'un identifiant à l'instrument médical d'intérêt 50 imagé sur l'image reçue. L'étape de classification est mise en œuvre par le calculateur 10 en interaction avec le produit programme d'ordinateur 12, c'est-à-dire est mise en œuvre par ordinateur.

Avantageusement, il est aussi déterminé une probabilité représentative d'un niveau de confiance en l'identifiant attribué à l'instrument médical d'intérêt 50. Cela permet d'alerter le personnel de santé d'une erreur potentielle dans l'identification effectuée. Par exemple, dans les cas où la probabilité est en-dessous d'un seuil prédéterminé (par exemple 85 %), il est considéré que l'identification est potentiellement erronée.

Le procédé d'identification comprend une étape 140 de caractérisation de l'instrument médical d'intérêt 50 en fonction de l'identifiant attribué. A l'issue de cette étape 140, il est ainsi obtenu au moins une caractéristique relative à l'instrument médical d'intérêt 50 imagé sur l'image reçue.

Par exemple, lorsque l'identifiant est associé à une donnée relative à une nature d'instrument médical, l'étape de caractérisation 140 comprend la détermination de la nature de l'instrument médical d'intérêt 50 imagé sur l'image reçu en fonction de l'identifiant attribué.

Par exemple, lorsque l'identifiant est associé à des recommandations d'utilisation de l'instrument médical d'intérêt 50, l'étape de caractérisation 140 comprend la détermination des recommandations d'utilisation de l'instrument médical d'intérêt 50 imagé sur l'image reçue en fonction de l'identifiant attribué.

Par exemple, lorsque l'identifiant est associé à un lien internet ou à une base de données à distance, correspondant à un support visuel relatif à l'utilisation de l'instrument médical d'intérêt 50, l'étape de caractérisation 140 comprend l'affichage du support visuel correspondant au lien internet ou à la base de données associé(e) à l'identifiant attribué.

Par exemple, lorsque l'identifiant est associé à une ou plusieurs caractéristiques structurelles, l'étape de caractérisation 140 comprend la détermination de la ou des caractéristiques structurelles de l'instrument médical d'intérêt 50 imagé sur l'image reçue en fonction de l'identifiant attribué.

L'étape de caractérisation 140 est, par exemple, mise en œuvre par le calculateur 10 en interaction avec le produit programme d'ordinateur 12, c'est-à-dire est mise en œuvre par ordinateur. Par exemple, les caractéristiques sont extraites d'une base de données en fonction de l'identifiant attribué.

En variante, l'étape de caractérisation 140 est mise en œuvre par un opérateur, tel qu'un personnel de santé (remplacement de l'instrument médical, injection de produits). L'opérateur va, par exemple, rechercher dans une base de données des caractéristiques correspondant à l'identifiant attribué.

Dans un exemple de mise en œuvre, le procédé d'identification comprend une étape 150 de manipulation de l'instrument médical d'intérêt 50 en fonction de la caractérisation effectuée. La manipulation est, par exemple, réalisée par un opérateur, tel qu'un personnel de santé.

Par exemple, en fonction de la caractérisation effectuée, l'instrument médical d'intérêt 50 sera manipulé différemment par le personnel de santé. Par exemple, les manipulations et produits injectés pourront différer entre un cathéter PICC et un cathéter Midline.

Ainsi, le présent procédé permet d'identifier un ou plusieurs instruments médicaux parmi un ensemble d'instruments médicaux référencés. Un tel procédé permet donc d'aider le personnel de santé à identifier un instrument avec un risque de confusion diminué.

L'identification des instruments médicaux d'intérêt permet, en outre, via la connaissance des recommandations d'utilisation et/ou des caractéristiques de l'instrument identifié, de :
- limiter les risques d'injection de molécules non adaptées à la voie d'abord de l'instrument,
- respecter les bonnes pratiques d'utilisation et d'entretien de l'instrument, et
- connaitre les incompatibilité de l'instrument avec d'autres dispositifs médicaux.

L'homme du métier comprendra que les modes de réalisation et variantes précédemment décrits peuvent être combinés pour former de nouveaux modes de réalisation pourvu qu'ils soient compatibles techniquement.

En particulier, en variante ou en complément facultatif des modes de réalisation précédents, l'identifiant attribué à l'instrument médical d'intérêt est associé à une base de données à distance par laquelle il est obtenu des informations d'utilisation de l'instrument médical. Cela permet d'avoir accès à des informations difficilement disponibles au domicile du patient, voire à l'hôpital. Or, ces informations sont cruciales pour l'utilisation en toute sécurité de l'instrument médical. En particulier, les deux exemples types suivants peuvent avoir des conséquences graves pour le patient si ces informations d'utilisation ne sont pas connues :
- 1^{er} exemple : Le débit maximal d'injection n'est pas respecté, ce qui entraîne la rupture du cathéter, et ainsi de potentiel extravasations.
- 2^{ème} exemple : Une mauvaise connaissance de la présence du cathéter dans le réseau veineux central ou périphérique est susceptible d'engendrer une injection de molécules veinotoxiques en périphérique détruisant le capital veineux du patient.

Ainsi, lorsque l'identifiant attribué à l'instrument médical d'intérêt est associé à une base de données à distance, le procédé permet d'avoir accès à de telles informations d'utilisation. Les informations d'utilisation sont typiquement spécifiques du fabricant (garantissant le bon usage) et/ou établies/complétées par les professionnels de santé par des données réelles d'utilisation. Ainsi, les informations d'utilisation sont par exemple :
- En provenance du fabricant (débit maximal), c'est-à-dire qu'on ne les trouve pas en faisant une recherche internet ou dans un article bibliographique, spécifiquement et avec certitude pour les références concernées, et/ou
- En provenance des professionnels de santé eux-mêmes (central ou périphérique).

Dans un exemple de mise en œuvre, les informations d'utilisation comprennent initialement une information par défaut, par exemple, une information relative à une caractéristique propre de l'instrument médical définie par le fabricant, par exemple, central ou périphérique. Ensuite, dans un second temps, les informations d'utilisation sont complétées par la personne manipulant l'instrument médical (professionnel de santé) en entrant dans la base de données des informations complémentaires du type : nom de la veine d'abord utilisée, longueur du cathéter implanté, la date d'implantation, etc. Ces informations complémentaires viennent, ainsi, compléter l'information par défaut. De préférence, les informations d'utilisation sont également complétées par tout utilisateur de l'instrument médical (professionnel de santé) en y intégrant des informations complémentaires telles que le type de traitement administré, ainsi que la qualité de l'injection (absence de douleur, rougeur, etc.) assurant ainsi la traçabilité de l'utilisation de l'instrument médical et sa sécurité.

En complément facultatif ou en variante, les informations d'utilisation sont archivées dans une base de données (dans une application ou mémoire) afin d'assurer le suivi du patient. Par exemple, une fois l'instrument médical identifié, le professionnel de santé peut retourner dans l'application pour retrouver les informations d'utilisation explicitées précédemment (fabricant et son propre suivi). Il pourra y retrouver tous les instruments médicaux utilisés scannés et leurs utilisations, assurant ainsi la traçabilité et la sécurité de ses actions.

De préférence, les informations d'utilisation sont propres à être extraites de ce suivi (par exemple l'ensemble des cathéters posés / utilisés par un opérateur) pour en faire un rapport statistique qui sera utilisable par les professionnels de santé par exemple au niveau : médico-légal ou pour du suivi de l'activité clinique ou encore pour le fabricant comme outil de suivi post marché (post CE) dans le cadre de la nouvelle réglementation MDR. Dans ce dernier cadre, l'objectif est de constituer une base de donnée de l'utilisation des instruments médicaux, notamment des cathéters.

En variante ou en complément facultatif, l'identifiant est associé à une information relative à l'impact environnemental et/ou à l'écoconception de l'instrument médical d'intérêt. Une telle information est par exemple délivrée par le fabricant. Une telle information comprend par exemple une notation de l'instrument médical d'intérêt. En particulier, une telle information comprend par exemple une ou plusieurs des données suivantes : l'impact carbone, les éventuels adjuvants rentrant dans la composition de l'instrument médical d'intérêt, et des informations relatives au recyclage (packaging) de l'instrument médical d'intérêt.

## Revendications

1. Procédé d'identification d'instruments médicaux, dit instruments médicaux d'intérêt (50), les instruments médicaux d'intérêt (50) étant des instruments destinés à être introduits dans le corps d'un patient ou à être mis en contact avec le corps d'un patient, ou étant des instruments destinés à être connectés à des instruments introduits dans le corps d'un patient ou en contact avec le corps d'un patient, chaque instrument médical d'intérêt (50) ayant une partie externe (54) à l'extérieur du corps du patient, le procédé comprenant les étapes suivantes :
a. la collection d'images de parties externes d'instruments médicaux d'intérêt (50), chaque image étant labellisée avec un identifiant de l'instrument médical d'intérêt (50) parmi un ensemble d'identifiants prédéfinis, les images collectées formant une base d'images d'entraînement, l'étape de collection étant mise en œuvre par ordinateur,
b. l'entraînement d'un modèle d'identification à partir de la base d'images d'entraînement pour obtenir un modèle d'identification entraîné pour attribuer un identifiant, parmi l'ensemble d'identifiants prédéfinis, à un instrument médical d'intérêt (50) à identifier en fonction d'une image de la partie externe (54) de l'instrument médical (50), l'étape d'entraînement étant mise en œuvre par ordinateur,
c. la réception d'une image de la partie externe (54) d'un instrument médical d'intérêt (50) à identifier, l'étape de réception étant mise en œuvre par ordinateur,
d. l'attribution, par le modèle de classification entraîné, d'un identifiant à l'instrument médical d'intérêt (50) imagé sur l'image reçu, l'étape de classification étant mise en œuvre par ordinateur, et
e. la caractérisation de l'instrument médical d'intérêt (50) en fonction de l'identifiant attribué,
dans lequel chaque identifiant est associé à un lien internet et/ou à une base de données à distance par lequel/laquelle il est obtenu un support visuel relatif à l'utilisation de l'instrument médical d'intérêt (50), l'étape de caractérisation comprenant l'affichage du support visuel correspondant au lien internet ou à la base de données associé(e) à l'identifiant attribué, le support visuel étant par exemple une vidéo, une image ou un article.

2. Procédé selon la revendication 1, dans lequel le procédé comprend une étape de manipulation de l'instrument médical d'intérêt (50) en fonction de la caractérisation effectuée.

3. Procédé selon la revendication 1 ou 2, dans lequel la base d'images d'entraînement est formée d'images d'instruments médicaux d'intérêt (50) d'au moins deux natures différentes, les au moins deux natures d'instruments médicaux (50) imagés sur les images de la base d'images d'entraînement, étant choisies parmi les natures suivantes : tube médical, cathéter veineux, cathéter artériel, cathéter de nutrition entéral, cathéter de nutrition parentéral, cathéter de drainage, cathéter péridural, cathéter systémique, cathéter veineux central à insertion périphérique, dit PICC, cathéter veineux périphérique, dit Midline, cathéter veineux central, dit CVC, cathéter ombilical, aiguille de Huber, système de fixation cutanée de tubes médicaux, pansement de recouvrement, valve, système clos et bouchon connecté à une embase d'un tube médical.

4. Procédé selon l'une quelconque des revendications 1 à 3, dans lequel chaque identifiant est associé à une donnée relative à une nature d'instrument médical, l'étape de caractérisation comprenant la détermination de la nature de l'instrument médical d'intérêt (50) imagé sur l'image reçue en fonction de l'identifiant attribué.

5. Procédé selon l'une quelconque des revendications 1 à 4, dans lequel chaque identifiant est associé à des recommandations d'utilisation de l'instrument médical d'intérêt (50), l'étape de caractérisation comprenant la détermination des recommandations d'utilisation de l'instrument médical d'intérêt (50) imagé sur l'image reçue en fonction de l'identifiant attribué.

6. Procédé selon la revendication 5, dans lequel les recommandations d'utilisation comprennent une ou plusieurs des recommandations suivantes :
a. la voie d'abord à utiliser pour l'introduction de l'instrument médical d'intérêt (50),
b. le débit maximal d'injection de liquide dans l'instrument médical d'intérêt (50),
c. la pression maximale de mise en défaut de l'instrument médical d'intérêt (50),
d. la fréquence de changement d'un éventuel pansement appliqué sur l'instrument médical d'intérêt (50),
e. la compatibilité de l'instrument médical d'intérêt (50) avec d'autres types d'instruments médicaux,
f. la compatibilité de l'instrument médical d'intérêt (50) avec des solutions médicamenteuses, et
g. la compatibilité de l'instrument médical d'intérêt (50) avec des examens médicaux prédéfinis, et le cas échéant, les conditions de réalisation des examens.

7. Procédé selon l'une quelconque des revendications 1 à 6, dans lequel chaque identifiant est associé à au moins une caractéristique structurelle de l'instrument médical d'intérêt (50), la ou au moins une caractéristique structurelle étant choisie parmi : la marque de l'instrument médical d'intérêt (50), le contact du fabricant légal de l'instrument médical d'intérêt (50), le ou les matériaux dans lesquels est réalisé l'instrument médical d'intérêt (50), le ou les éventuels matériaux potentiellement allergènes dans lesquels est réalisé l'instrument médical d'intérêt (50), la taille de l'instrument médical d'intérêt (50) et, lorsque l'instrument médical d'intérêt (50) est un cathéter ou un tube médical, le diamètre et la longueur initiale du cathéter ou du tube médical, le nombre de lumières du cathéter ou du tube médical, le volume mort de chaque lumière du cathéter ou du tube médical, les débits, par gravité ou par injection via une pompe ou un injecteur de produits de contraste, du cathéter ou du tube médical, l'étape de caractérisation comprenant la détermination de la ou des caractéristiques structurelles de l'instrument médical d'intérêt (50) imagé sur l'image reçue en fonction de l'identifiant attribué.

8. Procédé selon l'une quelconque des revendications 1 à 7, dans lequel chaque instrument médical d'intérêt (50) présente une partie interne (52) connectée à la partie externe (54), la partie interne (52) étant un tube ou une aiguille introduite dans le corps du patient, la partie externe (54) comprenant au moins un tube (59) et/ou à un connecteur à un tube (64 ; 70).

9. Procédé selon l'une quelconque des revendications 1 à 8, dans lequel l'identifiant attribué à l'instrument médical d'intérêt est associé à une base de données à distance par laquelle il est obtenu des informations d'utilisation de l'instrument médical.

10. Procédé selon la revendication 9, dans lequel les informations d'utilisation comprennent des données définies par le fabricant et/ou des données définies par un professionnel de santé lors de l'utilisation de l'instrument médical sur un patient.

11. Procédé selon la revendication 9 ou 10, dans lequel le procédé comprend une étape de mise à jour des informations d'utilisation de l'instrument médical pour un patient donné ou une utilisation donnée, les informations d'utilisation étant archivées de sorte à assurer un suivi du patient et/ou une traçabilité de l'instrument médical.

12. Procédé selon l'une quelconque des revendications 1 à 11, dans lequel l'identifiant est associé à une information relative à l'impact environnemental et/ou à l'écoconception de l'instrument médical d'intérêt.

13. Produit programme d'ordinateur comprenant des instructions de programme enregistrées sur un support lisible par ordinateur, pour l'exécution d'un procédé de d'identification selon l'une quelconque des revendications 1 à 12 lorsque le programme d'ordinateur est exécuté sur un ordinateur.

## Patentansprüche

1. Verfahren zum Identifizieren von medizinischen Instrumenten, sogenannten medizinischen Instrumenten von Interesse (50), wobei die medizinischen Instrumente von Interesse (50) Instrumente sind, die dazu bestimmt sind, in den Körper eines Patienten eingeführt oder mit dem Körper eines Patienten in Kontakt gebracht zu werden, oder Instrumente sind, die dazu bestimmt sind, mit Instrumenten verbunden zu werden, die in den Körper eines Patienten eingeführt oder mit dem Körper eines Patienten in Kontakt gebracht zu werden, wobei jedes medizinische Instrument von Interesse (50) einen äußeren Abschnitt (54) außerhalb des Körpers des Patienten aufweist, das Verfahren umfassend die folgenden Schritte:
a. Sammeln von Bildern von äußeren Abschnitten von medizinischen Instrumenten von Interesse (50), wobei jedes Bild mit einer Kennung des medizinischen Instruments von Interesse (50) aus einem Satz von vordefinierten Kennungen beschriftet ist, wobei die gesammelten Bilder eine Basis von Trainingsbildern bilden, wobei der Schritt eines Sammelns von einem Rechner implementiert wird,
b. Trainieren eines Identifikationsmodells anhand der Basis von Trainingsbildern, um ein Identifikationsmodell zu erlangen, das trainiert ist, um basierend auf einem Bild des äußeren Abschnitts (54) des medizinischen Instruments (50) eine Kennung aus dem Satz von vordefinierten Kennungen zu einem zu identifizierenden medizinischen Instrument (50) von Interesse zuzuordnen, wobei der Trainingsschritt von einem Rechner implementiert wird,
c. Empfangen eines Bilds des äußeren Abschnitts (54) eines medizinischen Instruments von Interesse (50), das identifiziert werden soll, wobei der Schritt eines Empfangens von einem Rechner implementiert wird,
d. Zuordnen, durch das trainierte Klassifizierungsmodell, einer Kennung zu dem medizinischen Instrument von Interesse (50), das auf dem empfangenen Bild abgebildet ist, wobei der Schritt eines Klassifizierens von einem Rechner implementiert wird, und
e. Charakterisieren des medizinischen Instruments von Interesse (50) basierend auf der zugeordneten Kennung,
wobei jede Kennung mit einem entfernten Internet-Link und/oder einer Datenbank verbunden ist, durch die ein visuelles Medium bezüglich der Verwendung des medizinischen Instruments von Interesse (50) erlangt wird, wobei der Schritt eines Charakterisierens das Anzeigen des visuellen Mediums umfasst, das dem Internet-Link oder der Datenbank entspricht, der/die mit der zugeordneten Kennung assoziiert ist, wobei das visuelle Medium beispielsweise ein Video, ein Bild oder ein Artikel ist.

2. Verfahren nach Anspruch 1, wobei das Verfahren einen Schritt eines Manipulierens des medizinischen Instruments von Interesse (50) abhängig von der durchgeführten Charakterisierung umfasst.

3. Verfahren nach Anspruch 1 oder 2, wobei die Basis von Trainingsbildern aus Bildern von medizinischen Instrumenten von Interesse (50) von mindestens zwei verschiedenen Arten gebildet ist, wobei die mindestens zwei Arten von medizinischen Instrumenten (50), die auf den Bildern der Basis von Trainingsbildern abgebildet sind, ausgewählt sind aus den folgenden Arten: medizinischer Schlauch, Venenkatheter, arterieller Katheter, enteraler Ernährungskatheter, parenteraler Ernährungskatheter, Drainagekatheter, Periduralkatheter, systemischer Katheter, peripherer zentraler Venenkatheter, PICC genannt, peripherer Venenkatheter, Midline genannt, zentraler Venenkatheter, ZVK genannt, Nabelkatheter, Huber-Nadel, Hautfixierungssystem für medizinische Schläuche, Abdeckverband, Ventil, geschlossenes System und Stopfen, der mit einem Ansatz eines medizinischen Schlauchs verbunden ist.

4. Verfahren nach einem der Ansprüche 1 bis 3, wobei jede Kennung mit Daten bezüglich einer Art eines medizinischen Instruments assoziiert ist, der Schritt eines Charakterisierens umfassend das Bestimmen der Art des medizinischen Instruments von Interesse (50), das auf dem empfangenen Bild abgebildet ist, basierend auf der zugeordneten Kennung.

5. Verfahren nach einem der Ansprüche 1 bis 4, wobei jede Kennung mit Empfehlungen für die Verwendung des medizinischen Instruments von Interesse (50) assoziiert ist, der Schritt eines Charakterisierens umfassend das Bestimmen der Empfehlungen für die Verwendung des medizinischen Instruments von Interesse (50), das auf dem empfangenen Bild abgebildet ist, abhängig von der zugeordneten Kennung.

6. Verfahren nach Anspruch 5, wobei die Empfehlungen für die Verwendung eine oder mehrere der folgenden Empfehlungen umfassen:
a. den Zugangsweg, der zum Einführen des medizinischen Instruments von Interesse (50) verwendet werden soll,
b. die maximale Injektionsrate von Flüssigkeit in das medizinische Instrument von Interesse (50),
c. der maximale Fehlerdruck des medizinischen Instruments von Interesse (50),
d. die Wechselhäufigkeit eines eventuell auf das medizinische Instrument von Interesse (50) aufgebrachten Verbands,
e. die Kompatibilität des medizinischen Instruments von Interesse (50) mit anderen Arten von medizinischen Instrumenten,
f. die Kompatibilität des medizinischen Instruments von Interesse (50) mit Arzneimittellösungen, und
g. die Kompatibilität des medizinischen Instruments von Interesse (50) mit vordefinierten medizinischen Untersuchungen und gegebenenfalls die Bedingungen für die Durchführung von Untersuchungen.

7. Verfahren nach einem der Ansprüche 1 bis 6, wobei jeder Identifikator mit mindestens einem Strukturmerkmal des medizinischen Instruments von Interesse (50) assoziiert ist, wobei das oder mindestens ein Strukturmerkmal ausgewählt ist aus: der Marke des medizinischen Instruments von Interesse (50), dem Kontakt des rechtmäßigen Herstellers des medizinischen Instruments von Interesse (50), dem Material oder den Materialien, aus denen das medizinische Instrument von Interesse (50) gefertigt ist, dem potenziell allergenen Material oder den potenziell allergenen Materialien, aus denen das medizinische Instrument von Interesse (50) gefertigt ist, der Größe des medizinischen Instruments von Interesse (50) und, wenn das medizinische Instrument von Interesse (50) ein Katheter oder eine medizinische Röhre ist, dem Durchmesser und der ursprünglichen Länge des Katheters oder der medizinischen Röhre, der Anzahl der Lumen des Katheters oder der medizinischen Röhre, dem Totvolumen jedes Lumens des Katheters oder der medizinischen Röhre, den Durchflussraten, durch Schwerkraft oder durch Injektion über eine Pumpe oder einen Kontrastmittelinjektor, des Katheters oder der medizinischen Röhre, der Schritt eines Charakterisierens umfassend das Bestimmen der strukturellen Eigenschaft(en) des medizinischen Instruments von Interesse (50), das auf dem empfangenen Bild abgebildet ist, basierend auf der zugeordneten Kennung.

8. Verfahren nach einem der Ansprüche 1 bis 7, wobei jedes medizinische Instrument von Interesse (50) einen inneren Abschnitt (52) aufweist, der mit dem äußeren Abschnitt (54) verbunden ist, wobei der innere Abschnitt (52) ein Rohr oder eine Nadel ist, das/die in den Körper des Patienten eingeführt wird, und der äußere Abschnitt (54) mindestens ein Rohr (59) umfasst und/oder einen Rohrverbinder (64; 70) aufweist.

9. Verfahren nach einem der Ansprüche 1 bis 8, wobei die mit dem medizinischen Instrument von Interesse zugeordnete Kennung mit einer entfernte Datenbank assoziiert ist, über die Informationen über die Verwendung des medizinischen Instruments erlangt werden.

10. Verfahren nach Anspruch 9, wobei die Verwendungsinformationen Daten umfassen, die von dem Hersteller definiert sind und/oder Daten, die von einem Angehörigen der Gesundheitsberufe bei der Verwendung des medizinischen Instruments an einem Patienten definiert sind.

11. Verfahren nach Anspruch 9 oder 10, wobei das Verfahren einen Schritt eines Aktualisierens der Verwendungsinformationen des medizinischen Instruments für einen bestimmten Patienten oder eine bestimmte Verwendung umfasst, wobei die Verwendungsinformationen archiviert werden, sodass eine Nachverfolgung des Patienten und/oder eine Rückverfolgbarkeit des medizinischen Instruments gewährleistet ist.

12. Verfahren nach einem der Ansprüche 1 bis 11, wobei die Kennung mit Informationen über die Umweltauswirkungen und/oder das Ökodesign des medizinischen Instruments von Interesse assoziiert ist.

13. Rechnerprogrammprodukt, umfassend Programmanweisungen, die auf einem rechnerlesbaren Medium aufgezeichnet sind, zum Ausführen eines Identifizierungsverfahrens nach einem der Ansprüche 1 bis 12, wenn das Rechnerprogramm auf einem Rechner ausgeführt wird.

## Claims

1. A method of identifying medical devices, called medical devices of interest (50), the medical devices of interest (50) being devices intended for being inserted into the body of a patient or for being brought into contact with the body of a patient, or being devices intended for being connected to devices inserted into or in contact with a patient's body, each medical device of interest (50) having an outer part (54) outside the patient's body, the method comprising the following steps:
a. the collection of images of outer parts of medical devices of interest (50), each image being labeled with an identifier of the medical device of interest (50) from a set of predefined identifiers, the collected images forming a training image database, the collection step being computer-implemented,
b. the training of an identification model from the training image database in order to obtain an identification model trained to assign an identifier, from the set of predefined identifiers, to a medical device of interest (50) to be identified according to an image of the outer part (54) of the medical device (50), the training step being computer-implemented,
c. the reception of an image of the outer part (54) of a medical device of interest (50) to be identified, the reception step being computer-implemented,
d. the assignment, by the trained classification model, of an identifier to the medical device of interest (50) imaged on the received image, the classification step being computer-implemented, and
e. characterizing the medical device of interest (50) according to the assigned identifier;
wherein each identifier is associated with an Internet link and/or a remote database through which visual support is obtained with regard to the use of the medical device of interest (50), the characterization step comprising the display of the visual medium corresponding to the Internet link or to the database associated with the assigned identifier, the visual medium being e.g. a video, an image or an article.

2. The method according to claim 1, wherein the method comprises a step of handling the medical device of interest (50) according to the characterization performed.

3. The method according to claim 1 or 2, the training image database consisting of images of medical devices of interest (50) of at least two different types, the at least two types of medical devices (50) imaged on the images of the training image database being selected from the following types: medical tube, venous catheter, arterial catheter, enteral nutrition catheter, parenteral nutrition catheter, drainage catheter, epidural catheter, systemic catheter, peripheral insertion central venous catheter, called PICC, peripheral venous catheter, called Midline, central venous catheter, called CVC, umbilical catheter, Huber needle, cutaneous securement system for medical tubes, covering dressing, valve, closed system and stopper connected to a hub of a medical tube.

4. The method according to any of claims 1 to 3, wherein each identifier is associated with data relating to a type of medical device, the characterization step comprising determination of the type of the medical device of interest (50) imaged on the received image according to the assigned identifier.

5. The method according to any of claims 1 to 4, wherein each identifier is associated with recommendations for use of the medical device of interest (50), the characterization step comprising the determination of recommendations for use of the medical device of interest (50) imaged on the received image according to the assigned identifier.

6. The method according to claim 5, wherein the recommendations for use comprises one or a plurality of the following recommendations:
a. the first route of medication to be used for the insertion of the medical device of interest (50),
b. the maximum rate of injection of liquid into the medical device of interest (50),
c. the maximum fault pressure of the medical device of interest (50),
d. the frequency of change of a dressing, if any, applied to the medical device of interest (50),
e. the compatibility of the medical device of interest (50) with other types of medical devices,
f. the compatibility of the medical device of interest (50) with medicinal solutions, and
g. the compatibility of the medical device of interest (50) with pre-defined medical examinations and, where appropriate, the conditions for carrying out the examinations.

7. The method according to any of claims 1 to 6, wherein each identifier is associated with at least one structural feature of the medical device of interest (50), the or at least one structural feature being chosen from: the brand name of the medical device of interest (50), the contact details of the legal manufacturer of the medical device of interest (50), the material or materials from which the medical device of interest (50) is made, the potential allergenic material(s), if any, from which the medical device of interest (50) is made, the size of the medical device of interest (50) and, when the medical device of interest (50) is a catheter or a medical tube, the diameter and the initial length of the catheter or the medical tube, the number of lumens in the catheter or the medical tube, the dead volume of each lumen of the catheter or of the medical tube, the flow rates, by gravity or by injection, via a pump or an injector of contrast substances, of the catheter or of medical tube, the characterization step comprising the determination of the structural feature(s) of the medical device of interest (50) imaged on the received image according to the assigned identifier.

8. The method according to any of claims 1 to 7, wherein each medical device of interest (50) has an inner portion (52) connected to the outer portion (54), the inner portion (52) being a tube or a needle inserted into the patient's body, the outer part (54) comprising at least one tube (59) and/or a connector to a tube (64; 70).

9. The method according to any of claims 1 to 8, wherein the identifier assigned to the medical device of interest is associated with a remote database from which information on the use of the medical device is obtained.

10. The method according to claim 9, wherein the information on use comprise data defined by the manufacturer and/or data defined by a healthcare professional when using the medical device on a patient.

11. The method according to claim 9 or 10, wherein the method comprises a step of updating the information for use of the medical device for a given patient and/or a given use, the information for use being archived so as to ensure a patient follow-up and/or a traceability of the medical device.

12. The method according to any of claims 1 to 11, wherein the identifier is associated with information with regard to the environmental impact and/or the eco-design of the medical device of interest.

13. A computer program product including program instructions stored on a computer-readable storage medium implementing steps of the identification method according to any of claims 1 to 12 when the computer program is executed on a computer.
